# NEUE EUROPÄISCHE PATENTSCHRIFT

(11) **EP 1 033 113 B2**
(45) Veröffentlichungstag und Bekanntmachung des Hinweises auf die Entscheidung über den Einspruch: **23.01.2008**
(45) Hinweis auf die Patenterteilung: 19.05.2004
(21) Anmeldenummer: 00103133.5
(22) Anmeldetag: 16.02.2000
(51) Int. Cl.: A61B 19/00

(54) **Verfahren und Vorrichtung zur Korrelation der tatsächlichen Lage eines Markierungselementes mit den durch ein Abbildungsverfahren erhaltenen Positionsdaten**
Method and apparatus for correlating the actual position of a marker with position data obtained by imaging
Méthode et dispositif pour corréler la position réelle d'un marqueur avec des données obtenues par imagerie

(30) Priorität: 01.03.1999 DE 19908844
(43) Veröffentlichungstag der Anmeldung: 06.09.2000
(73) Patentinhaber: AESCULAP AG & Co. KG, 78532 Tuttlingen (DE)
(72) Erfinder: Kozak, Josef, D-78532 Tuttlingen (DE)
(74) Vertreter: Boehme, Ulrich

(56) Entgegenhaltungen:
- DE-C2- 19 639 615
- US-A- 5 315 630
- US-A- 5 394 457
- US-A- 5 446 548
- US-A- 5 469 847
- US-A- 5 636 255
- US-A- 5 868 675

## Beschreibung

Die Erfindung betrifft ein Verfahren zur Korrelation der tatsächlichen Lage eines an einem Körper fixierten Markierungselementes mit den Positionsdaten des Markierungselementes, die sich aus schichtweise den Körper und die Markierungselemente abbildenden Abbildungsverfahren ergeben, bei dem man die Lage des Markierungselementes im Raum mittels eines am Markierungselement anlegbaren Tastinstrumentes abtastet, dessen räumliche Positionierung bestimmt und diese Positionsdaten mit den Positionsdaten korreliert, die sich aus dem Abbildungsverfahren für das Markierungselement ergeben.

Weiterhin betrifft die Erfindung ein Markierungselement zur Durchführung dieses Verfahrens.

Es sind eine Anzahl von abbildenden Verfahren bekannt, mit denen es gelingt, Körperstrukturen im Inneren des menschlichen oder tierischen Körpers schichtweise festzustellen, derartige Verfahren sind beispielsweise die Computertomographie oder die Kernspinresonanz. Um die dadurch gewonnenen Bilddaten, die Positionsangaben über die jeweiligen Körperteile abgeben, beispielsweise bei einer Operation mit den tatsächlichen Positionsdaten dieser Körperteile auf dem Operationstisch vergleichen zu können, ist es notwendig, die tatsächlichen Positionsdaten mit den gemessenen Positionsdaten der Abbildung zu korrelieren. Es ist bekannt, zu diesem Zweck am Körper Markierungselemente anzubringen, beispielsweise Knochenschrauben, die bei dem Abbildungsverfahren mit abgebildet werden. Dadurch ergibt sich die Möglichkeit, die tatsächlichen Positionsdaten dieser Markierungselemente im Raum mit den durch das Abbildungsverfahren bestimmten Positionsdaten zu korrelieren.

Schwierig bei diesem Verfahren ist allerdings, daß bei der Bestimmung der Positionsdaten der abgebildeten Markierungselemente nur eine begrenzte Genauigkeit erreicht werden kann, dies liegt einmal daran, daß die Genauigkeit durch den Abstand der Schichten begrenzt wird, in denen entsprechende Abbildungen vorliegen, zum anderen werden durch die Abbildungsverfahren nur Schnitte durch die Markierungselemente abgebildet, die entsprechend der jeweiligen Lage der Markierungselemente im Körper in völlig unvorhersehbarer Richtung durch das Markierungselement laufen und daher nur die ungefähre Positionierung des Markierungselementes angeben können, nicht aber die genaue Positionierung.

Bei der Korrelierung kann man dann zwar mit Hilfe eines Tastinstrumentes, welches an dem Markierungselement in bestimmter Weise anlegbar ist und dessen Position durch geeignete Meßvorrichtungen im Raum relativ genau zu bestimmen ist, beispielsweise unter Zuhilfenahme von Ultraschallsensoren oder Infrarotsensoren, die tatsächliche Position des Markierungselementes relativ genau bestimmen, nicht aber die genaue Positionierung des Markierungselementes relativ zu den anderen abgebildeten Körperteilen. Dieser Fehler geht auch in die Korrelation ein und führt dazu, daß letztendlich bei der Korrelation dieser Positionsdaten die erreichbare Genauigkeit in der Größenordnung einiger Millimeter liegt und daß Fehler in der Größenordnung von zwischen 1 mm und 4 mm in Kauf genommen werden müssen.

In der DE 196 39 615 C2 ist ein Markierelement beschrieben, das kugelförmig ausgebildet ist und eine trichterförmige Vertiefung aufweist, deren Spitze im Kugelmittelpunkt liegt. Es ist dadurch möglich, mit einem navigierten Tastinstrument diesen Kugelmittelpunkt mittels des Navigationssystems zu bestimmen. Die Geometrie des Markierelementes muß dazu vorbekannt sein.

US-A-5 868 675 offenbart ein Verfahren und ein Markierungselement gemäß den Oberbegriffen der Ansprüche 1 und 4.

Es ist Aufgabe der Erfindung, ein Verfahren anzugeben, mit dem diese Korrelationsgenauigkeit verbessert werden kann.

Diese Aufgabe wird bei einem Verfahren der eingangs beschriebenen Art erfindungsgemäß dadurch gelöst, daß man am Markierungselement eine Einführöffnung für das Tastinstrument vorsieht, die am Schwerpunkt des Markierungselementes endet, so daß das Tastinstrument so einführbar ist, daß es relativ zum Schwerpunkt des Markierungselementes eine definierte Positionierung einnimmt, daß man in den durch das Abbildungssystem erzeugten Schichtabbildungen den Flächenschwerpunkt der in der jeweiligen Schichtabbildung abgebildeten Querschnittsfläche des Markierungselementes bestimmt, daß man für jedes Markierungselement aus den Koordinaten der Flächenschwerpunkte dieser Querschnittsflächen in verschiedenen Schichtabbildungen die Koordinaten des räumlichen Schwerpunktes des Markierungselementes bestimmt und daß man die tatsächlichen Positionsdaten des Schwerpunktes des Markierungselementes, die man durch die Positionsdaten des Tastinstrumentes erhält, mit den Positionsdaten des Schwerpunktes desselben Markierungselementes korreliert, die man aus den Schichtabbildungen erhält.

Bei diesem Verfahren wird also zunächst das Tastelement relativ zum Schwerpunkt des Markierungselementes in eine genau bestimmte Position gebracht, so daß man aus der Positionsbestimmung des Tastinstrumentes exakt die Lage des Schwerpunktes des Markierungselementes erhalten kann.

Bei den Schichtabbildungen des Markierungselementes erhält man in verschiedenen benachbarten Schichtabbildungen unterschiedliche Querschnitte des abgebildeten Markierungselementes, und hier wird in jeder Schichtabbildung der Flächenschwerpunkt der Abbildung des Markierungselementes berechnet, dies läßt sich anhand der Grauwerte der Abbildung ohne weiteres realisieren. Nach Berechnung der Flächenschwerpunkte in verschiedenen Schichtabbildungen kann aus diesen Daten dann der räumliche Schwerpunkt des Markierungselementes berechnet werden, und dieser kann auch in beliebiger Weise zwischen den Schichten der Schichtabbildungen liegen, man erhält auf diese Weise also sehr genaue Positionsdaten für den Schwerpunkt des Markierungselementes, ohne dazu die genaue geometrische Anordnung des Markierungselementes im Körper bestimmen zu müssen.

Die Positionsdaten des in dieser Weise berechneten räumlichen Schwerpunktes können nunmehr mit den tatsächlichen Positionsdaten des Schwerpunktes des Markierungselementes korreliert werden, die durch das Tastinstrument erfaßt worden sind, und auf diese Weise erhält man eine sehr genaue Korrelation der tatsächlichen Positionsdaten des Markierungselementes und der aus den Abbildungsverfahren entnommenen Positionsdaten des Markierungselementes.

Wenn auf diese Weise eine größere Anzahl von Markierungselementen am Patienten vorgesehen und berücksichtigt werden, kann eine sehr exakte räumliche Korrelation zwischen den Positionsdaten des Patienten auf dem Operationstisch und den Positionsdaten des Patienten im Abbildungssystem hergestellt werden.

Die Einführöffnung kann so ausgebildet sein, daß das Tastinstrument nicht bis in den Schwerpunkt des Markierungselementes reicht, wenn die Anordnung des Tastinstrumentes im eingeführten Zustand relativ zum Schwerpunkt jedoch bekannt ist, kann ohne weiteres aus der Lage des Tastinstrumentes auch die Lage des Schwerpunktes berechnet werden.

Da die Einführöffnung am Schwerpunkt des Markierungselementes endet, wird die Spitze des Tastinstrumentes automatisch den Schwerpunkt markieren, wenn das Tastinstrument in die Einführöffnung eingeführt ist.

Insbesondere kann man ein Markierungselement mit einer kegelförmigen Einführungsöffnung verwenden, deren Spitze im Schwerpunkt des Markierungselementes angeordnet ist. Dadurch ist es sehr einfach, ein zum Beispiel nadelförmiges Tastinstrument so an das Markierungselement anzulegen, daß die Spitze in jedem Falle genau den Schwerpunkt markiert.

Günstig ist es weiterhin, wenn man ein Markierungselement in Form einer kreiszylindrischen Scheibe benutzt. Eine derartige geometrische Form erleichtert die Bestimmung einmal des Flächenschwerpunktes aus den Schichtabbildungen und zum anderen auch die Berechnung des räumlichen Schwerpunktes aus den Positionsdaten der Flächenschwerpunkte.

Der Erfindung liegt weiterhin die Aufgabe zugrunde, ein Markierungselement zur Durchführung des beschriebenen Verfahrens zu schaffen. Diese Aufgabe wird bei einem Markierungselement erfindungsgemäß dadurch gelöst, daß es eine Einführöffnung aufweist, die an dessen Schwerpunkt endet.

Insbesondere kann vorgesehen sein, daß die Einführöffnung die Form eines Kegels hat, dessen Spitze im Schwerpunkt des Markierungselementes angeordnet ist.

Günstig ist es, wenn das Markierungselement die Form einer kreiszylindrischen Scheibe aufweist.

Die nachfolgende Beschreibung bevorzugter Ausführungsformen der Erfindung dient im Zusammenhang mit der Zeichnung der näheren Erläuterung. Es zeigen:
- Figur 1:: Die Anordnung einer größeren Anzahl von Markierungselementen am menschlichen Körper;
- Figur 2:: Eine Längsschnittansicht eines Markierungselementes mit kegelförmiger Einführöffnung und einem in diese eingesetzten Tastinstrument.

Zur Bestimmung der Position eines Patienten werden an diesem mehrere Markierungselemente 1 befestigt, beispielsweise durch Aufkleben auf die Haut, durch Einschrauben von Knochenschrauben in Knochenteile etc.

Diese Markierungselemente sind an verschiedenen Stellen des Körpers festgelegt, und durch Bestimmung der räumlichen Koordinaten aller Markierungselemente kann die Lage des Patienten im Raum eindeutig beschrieben werden.

Die hier verwendeten Markierungselemente 1 sind kreiszylindrische Scheiben aus einem Material, das im Abbildungsverfahren sichtbar ist, also beispielsweise bei Verwendung eines Computertomographieverfahrens ein Material, das Röntgenstrahlen absorbiert, im Falle einer Kernspintomographie ein Material, welches Energie aus dem Mikrowellenfeld des Kernspintomographen absorbiert.

In seinem mittleren Bereich ist in das Markierungselement 1 eine kegelförmige Vertiefung 2 eingearbeitet, deren Spitze 3 im räumlichen Schwerpunkt S des Markierungselementes 1 endet.

In diese Vertiefung 2 ist ein Tastinstrument 4 mit einer Tastspitze 5 einführbar, so daß die Tastspitze 5 bei eingeführtem Tastinstrument 4 an der Spitze 3 der kegelförmigen Vertiefung 2 angeordnet ist, also im Schwerpunkt S des Markierungselementes 1.

Das Tastinstrument 4 trägt - in der Zeichnung nur schematisch dargestellt - mehrere Positionsbestimmungselemente 6, beispielsweise Ultraschallsensoren oder Infrarotdioden, die eine Schall- bzw. eine elektromagnetische Strahlung aussenden, die von entsprechenden Empfängern 7 einer Meßeinrichtung aufgenommen werden. Diese an sich bekannte Vorrichtung bestimmt über die Laufzeiten der Strahlungen zwischen den Positionsbestimmungselementen 6 und den Empfängern 7 die Position des Tastinstrumentes 4 relativ zu den Empfängern 7, und gestattet damit eine genaue Bestimmung der Positionsdaten der Tastspitze 5 relativ zu den Empfängern 7, also relativ zu einem ortsfesten System. Damit werden auch die Positionsdaten des Schwerpunktes S des Markierungselementes 1 relativ zu den Empfängern 7 bestimmt.

Diese Daten werden einer Datenverarbeitungsanlage 8 zugeführt, in welche außerdem die Schichtabbildungsdaten eingespeichert sind, die von der zu behandelnden Person durch ein schichtweise arbeitendes Abbildungsverfahren gewonnen worden sind. Derartige Verfahren können beispielsweise die Computertomographie oder die Kernspinresonanztomographie sein, und mit Hilfe derartiger Verfahren kann beispielsweise vor Beginn einer Operation der Patient untersucht worden sein, so daß die anatomischen Daten des Patienten auf diese Weise durch eine größere Anzahl von Schichtabbildungen zur Verfügung stehen. Bei diesen Schichtabbildungen werden auch die Markierungselemente 1 mit abgebildet, die vor der entsprechenden Untersuchung eingesetzt worden sind.

Die Datenverarbeitungsanlage 8 bestimmt in den einzelnen Schichtabbildungen die Querschnittsfläche, die jeweils von einem Markierungselement 1 eingenommen wird. Diese Querschnittsfläche ist ohne weiteres erkennbar und hängt von den Absorptionseigenschaften des Materials der Markierungselemente 1 ab. Wenn diese Absorptionseigenschaften spezifisch sind, kann die Querschnittsfläche der Markierungselemente 1 ohne weiteres von anderen Körperstrukturen unterschieden werden, so daß visuell oder auch automatisch die Querschnittsfläche eines Markierungselementes 1 in verschiedenen benachbarten Schichtabbildungen festgestellt werden kann.

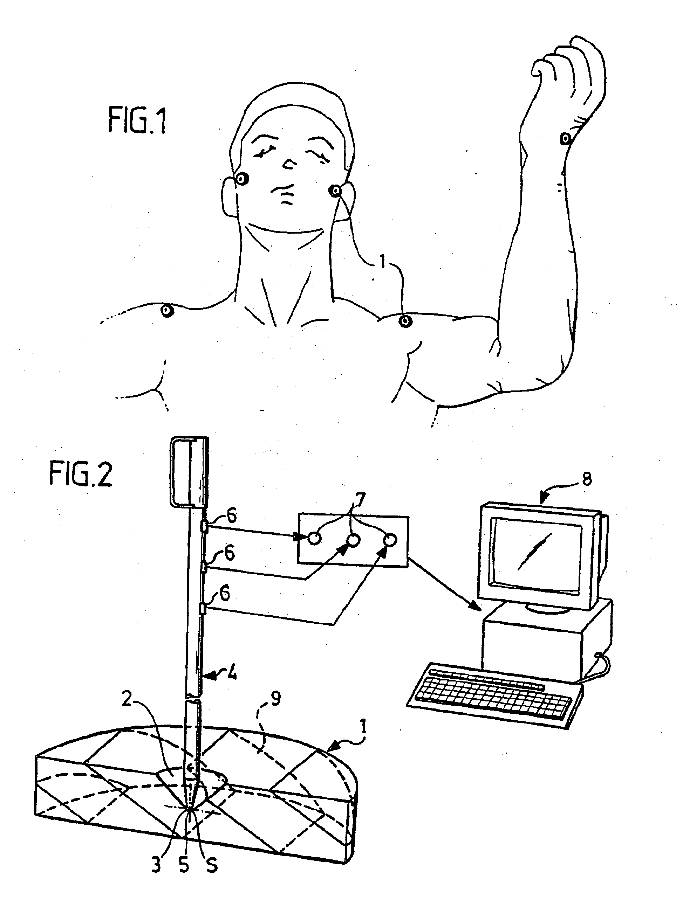

In jeder einzelnen Schichtabbildung berechnet die Datenverarbeitungsanlage 8 den Flächenschwerpunkt der Querschnittsfläche eines bestimmten Markierungselementes 1, und aus den Positionsdaten dies Flächenschwerpunkte lassen sich mathematisch die Positionsdaten des räumlichen Schwerpunktes des Markierungselementes 1 bestimmen, und zwar mit einer sehr großen Genauigkeit. In Figur 2 sind schematisch die entsprechenden Querschnittsflächen 9 des Markierungselementes dargestellt, die sich bei einer bestimmten Orientierung der Schichtabbildungen ergeben und die sich als Durchstoßflächen des Markierungselementes durch die Abbildungsebenen darstellen. In dem dargestellten Ausführungsbeispiel wird das Markierungselement durch vier benachbarte Abbildungen geschnitten, so daß sich in vier benachbarten Abbildungen Querschnittsflächen 9 des Markierungselementes 1 zeigen. In jeder dieser Querschnittsflächen 9 wird der Flächenschwerpunkt rechnerisch bestimmt, und aus diesen Positionsdaten wird dann ebenfalls rechnerisch der räumliche Schwerpunkt bestimmt.

Auf diese Weise sind aus den Abbildungen die Positionsdaten des Schwerpunktes jedes Markierungselementes 1 mit sehr hoher Genauigkeit zu bestimmen, und diese Positionsdaten können jetzt mit den Positionsdaten derselben Markierungselemente 1 verglichen werden, die durch das Tastinstrument 4 in der beschriebenen Weise bestimmt worden sind. Damit ist eine sehr exakte Korrelation der momentanen, tatsächlichen Position des Patienten im Raum mit den durch das Abbildungsverfahren bestimmten Positionsdaten möglich, und diese Korrelation, die auch als Eichung oder Registrierung bezeichnet wird, kann auch während einer Operation zu Kontrollzwecken jederzeit leicht wiederholt werden. Es genügt dazu, die Vertiefungen 2 der Markierungselemente 1 mit dem Tastinstrument 4 nacheinander anzufahren und damit die momentane Positionierung des Schwerpunktes jedes Markierungselementes 1 erneut zu bestimmen, diese Daten können dann mit den aus dem Abbildungssystem gewonnenen Positionsdaten der Schwerpunkte der Markierungselemente 1 verglichen werden, die Eichung kann also auf diese Weise wiederholt werden.

Sehr wesentlich bei dem beschriebenen Verfahren ist die Tatsache, daß die Bestimmung der Schwerpunktskoordinaten aus dem Abbildungsverfahren vollautomatisch ablaufen kann, so daß visuelle Fehler vermieden werden können. Außerdem wird das Verfahren dadurch erheblich beschleunigt.

## Patentansprüche

1. Verfahren zur Korrelation der tatsächlichen Lage eines an einem Körper fixierten Markierungselementes mit den Positionsdaten des Markierungselementes, die sich aus schichtweise den Körper und die Markierungselemente abbildenden Abbildungsverfahren ergeben, bei denen man die Lage des Markierungselementes im Raum mittels eines am Markierungselement anlegbaren Tastinstrumentes abtastet, dessen räumliche Positionierung bestimmt und diese Positionsdaten mit den Positionsdaten korreliert, die sich aus dem Abbildungsverfahren für das Markierungselement ergeben, **dadurch gekennzeichnet, daß** man am Markierungselement eine Einführöffnung für das Tastinstrument vorsieht, die am Schwerpunkt des Markierungselementes endet, so daß das Tastinstrument so einführbar ist, daß es relativ zum Schwerpunkt des Markierungselementes eine definierte Positionierung einnimmt, daß man in den durch das Abbildungssystem erzeugten Schichtabbildungen den Flächenschwerpunkt der in der jeweiligen Schichtabbildung abgebildeten Querschnittsfläche des Markierungselementes bestimmt, daß man für jedes Markierungselement aus den Koordinaten der Flächenschwerpunkte dieser Querschnittsflächen in verschiedenen Schichtabbildungen die Koordinaten des räumlichen Schwerpunktes des Markierungselementes bestimmt und daß man die tatsächlichen Positionsdaten des Schwerpunktes des Markierungselementes, die man durch die Positionsdaten des Tastinstrumentes erhält, mit den Positionsdaten des Schwerpunktes desselben Markierungselementes korreliert, die man aus den Schichtabbildungen erhält.

2. Verfahren nach Anspruch 1, **dadurch gekennzeichnet, daß** man ein Markierungselement mit einer kegelförmigen Einführöffnung verwendet, deren Spitze im Schwerpunkt des Markierungselementes angeordnet ist.

3. Verfahren nach Anspruch 1 oder 2, **dadurch gekennzeichnet, daß** man ein Markierungselement in Form einer kreiszylindrischen Scheibe benutzt.

4. Markierungselement zur Durchführung des Verfahrens nach einem der Ansprüche 1 bis 3, **dadurch gekennzeichnet, daß** es eine Einführöffnung (2) aufweist, die an dessen Schwerpunkt (S) endet.

5. Markierungselement nach Anspruch 4, **dadurch gekennzeichnet, daß** die Einführöffnung (2) die Form eines Kegels hat, dessen Spitze (3) im Schwerpunkt (S) des Markierungselementes (1) angeordnet ist.

6. Markierungselement nach Anspruch 4 oder 5, **dadurch gekennzeichnet, daß** das Markierungselement (1) die Form einer kreiszylindrischen Scheibe aufweist.

## Claims

1. A method of correlating the actual position of a marker fixed to a body with the positional data of the marker obtained from imaging processes which depict the body and the markers layer by layer and in which the three-dimensional position of the marker is scanned by means of a scanning instrument which can be applied to the marker, the spatial position of the scanning instrument is determined, and these positional data are correlated with the positional data obtained from the imaging process for the marker, **characterised in that** the marker is provided with an insertion opening for the scanning instrument, which opening ends at the centre of gravity of the marker so that the scanning instrument may be inserted so that it adopts a defined position relative to the centre of gravity of the marker, **in that**, in the layer images generated by the imaging system, the two-dimensional centre of gravity of the cross-sectional area of the marker depicted in the respective layer image is determined, **in that** the co-ordinates of the three-dimensional centre of gravity of the marker are determined for each marker from the co-ordinates of the two-dimensional centres of gravity of said cross-sectional areas in different layer images, and **in that** the actual positional data of the centre of gravity of the marker, obtained from the positional data of the scanning instrument, are correlated with the positional data of the centre of gravity of the same marker obtained from the layer images.

2. A method according to claim 1, **characterised in that** a marker with a conical insertion opening is used, the tip of which is arranged in the centre of gravity of the marker.

3. A method according to claim 1 or 2, **characterised in that** a marker in the form of a circular cylindrical disc is used.

4. A marker for carrying out the method according to any one of claims 1 to 3, **characterised in that** it has an insertion opening (2) ending at its centre of gravity (S).

5. A marker according to claim 4, **characterised in that** the insertion opening (2) is in the shape of a cone, the tip (3) of which is arranged in the centre of gravity (S) of the marker (1).

6. A marker according to claim 4 or 5, **characterised in that** the marker (1) is in the form of a circular cylindrical disc.

## Revendications

1. Procédé de corrélation de la position effective d'un marqueur fixé sur un corps avec les données de position du marqueur, qui résultent d'un procédé d'imagerie représentant par couches le corps et les marqueurs, dans lequel on explore la position du marqueur dans l'espace au moyen d'un palpeur applicable au marqueur, on détermine le positionnement spatial de celui-ci et on corrèle les données de position avec les données de position, qui résultent du procédé d'imagerie pour le marqueur, **caractérisé en ce qu'**on prévoit sur le marqueur une ouverture d'introduction pour le palpeur, qui se termine dans le centre de gravité du marqueur, de sorte que le palpeur peut être introduit, de sorte qu'il adopte, par rapport au centre de gravité du marqueur, un positionnement défini, **en ce qu'**on détermine dans les images de couches générées par le système d'imagerie, le centre de gravité de la surface en coupe représentée dans l'image de couche respective du marqueur, **en ce qu'**on détermine pour chaque marqueur à partir des coordonnées du centre de gravité de ces surfaces en coupe dans différentes images de couches les coordonnées du centre de gravité spatial du marqueur et **en ce qu'**on corrèle les données de position effectives du centre de gravité du marqueur, que l'on obtient grâce aux données de position du palpeur, avec les données de position du centre de gravité du même marqueur, que l'on obtient à partir des images de couches.

2. Procédé selon la revendication 1, **caractérisé en ce qu'**on utilise un marqueur avec une ouverture d'introduction conique dont la pointe est disposée au centre de gravité du marqueur.

3. Procédé selon la revendication 1 ou 2, **caractérisé en ce qu'**on utilise un marqueur sous la forme d'un disque cylindrique circulaire.

4. Marqueur en vue de la mise en oeuvre du procédé selon l'une des revendications 1 à 3, **caractérisé en ce qu'**il présente une ouverture d'introduction (2), qui se termine au centre de gravité (S) de celui-ci.

5. Marqueur selon la revendication 4, **caractérisé en ce que** l'ouverture d'introduction (2) présente une forme conique, dont la pointe (3) est disposée au centre de gravité (S) du marqueur (1).

6. Marqueur selon la revendication 4 ou 5, **caractérisé en ce que** le marqueur (1) présente la forme d'un disque cylindrique circulaire.
